# EUROPEAN PATENT SPECIFICATION

(11) **EP 1 611 867 B1**
(45) Date of publication and mention of the grant of the patent: **03.12.2008**
(21) Application number: 05018644.4
(22) Date of filing: 24.09.2001
(51) Int. Cl.: A61F 2/82

(54) **Intravascular stent**
Intravaskulärer Stent
Stent intravasculaire

(30) Priority: 23.09.2000 US 235167 P
(43) Date of publication of application: 04.01.2006
(62) Divisional of application: 01977156.7
(73) Proprietor: Boston Scientific Scimed, Inc., Maple Grove, MN 55311 (US)
(72) Inventor: Jang, David G., Redlands, CA 92374 (US)
(74) Representative: Hauck Patent- und Rechtsanwälte

(56) References cited:
- WO-A-00/02502
- WO-A-00/62710
- WO-A-02/24109
- WO-A-97/40783
- WO-A-99/15107
- WO-A-99/38457
- WO-A-99/40876

## Description

### BACKGROUND OF THE INVENTION

### Field of Invention:

This invention relates to intravascular stents in general, and more particularly to intracoronary stents.

### Description of the Related Art

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unfulfilled gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies indicate evidence of some suppression of restenosis after stent implantation when certain growth blocking or pharmaceutical agents coated the stent. In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, a good vessel coverage, a good internal surface modulation without tulips (i. e., sharp metal loop projections that resemble fish scale phenomena), an optimal vessel conformability, a low metal fraction, and so forth. If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the postdelivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor (inner and/or outer) surface modulation due to large numbers of tulips directed to both ends of the stent. Most of the current flexible stents are designed to effect flexibility by using fewer or a minimal number of connecting struts, handicapping the vessel coverage, surface modulation and tissue prolapse defects.

On the other hand, a stent that is designed for good vessel coverage and ideal cell size tends to be inflexible when such a stent is being delivered to a vessel lesion. A lack of flexibility during stent delivery is a very critical issue; a stiff stent often cannot be delivered to a needed location inside a blood vessel because such a stent cannot navigate through a tortuous and small vessel lumen.

WO 99/38457 shows a tubular stent consisting of horizontal expansion struts and contralaterally attached diagonal-connectors. The stent includes a plurality of expansion struts defining a first expansion column, and a plurality of expansion struts defining a second expansion column. Each expansion column includes first and second expansion struts joined at their ends by joining struts. Some of the expansion struts have a stepped distal or proximal portion. A connecting strut column is formed of a plurality of serial connecting struts. The connecting strut column couples the first expansion column to the second expansion column. The connecting struts have a stair-step configuration and are connected to the first and second expansion struts in the vicinity of their proximal and distal ends.

WO 99/40876 shows a stent having a first column expansion strut pair that includes a first expansion strut, a second expansion strut and a joining strut. A second column expansion strut pair has a first expansion strut, a second expansion strut and a joining strut. A plurality of first serial connecting struts form a first serial connecting strut column. The plurality of first serial connecting struts couple the first expansion column to the second expansion column. The first serial connecting strut ipsilaterally couples the second expansion strut of the first expansion column to the second expansion strut of the second expansion column.

WO 97/40783 shows a stent with a plurality of first expansion strut pairs forming a first expansion column, a plurality of second expansion strut pairs forming a second expansion column and a plurality of first connecting struts forming a first connecting strut column that couples the first expansion column to the second expansion column. The expansion struts do not have a stair-step segment at a proximal end.

WO 00/02502 shows a stent comprising a plurality of spaced band-like elements and intersecting links. The band-like elements have a generally serpentine configuration to provide continuous waves of generally sinusoidal character to each band-like element. The expansion struts do not have a stair-step segment at a proximal end.

WO 99/15107 shows a stent including first and second expansion columns and a first connecting strut column formed of a plurality of first connecting struts coupling the first expansion column to the second expansion column. The struts of the expansion columns do not have a stair-step segment at a proximal end.

There is a need for a vascular stent that is very flexible for delivery and with good vessel coverage when deployed.

### SUMMARY OF THE INVENTION

Various embodiments of a stent include a combination of maximum possible flexibility and conformability in the stent, full vessel coverage with optimal metal fraction, evenly expanding stent struts, excellent radial strength and radiopacity, and smooth surface modulations in both delivery and deployed phases of the stent life cycle. To arrive at these goals, many innovative new configurations are added to the expansion and connecting strut designs of the stent. Expansion strut design is largely responsible for radial strength and radiopacity, while connecting strut design is largely responsible for flexibility and smooth surface modulations. Full vessel coverage and uniform stent expansion are largely from interaction between expansion and connecting struts.Various embodiments of the stent demonstrate a balance among these multiple qualities, using smart expansion struts and flexible connecting struts in a seamlessly integrated stent network.

The embodiments of the stent are specifically designed to be both very flexible and fully cover vessel surface inside the vascular lumen. The stent can have both characteristics of vessel coverage and flexibility, particularly for coronary use.

Various embodiments of a stent are well designed for both the delivery phase and the deployed phase of the stent life cycle. Both flexibility and good vessel coverage are in a right balance in different embodiments in the stent. Numerous embodiments of the stent include certain configurations in expansion and connecting struts of the stent.

The stent includes a first expansion column, a second expansion column, and a first connecting strut column. The first expansion column and the second expansion column each include individual expansion struts forming a plurality of expansion strut pairs. Two adjacent expansion strut pairs share a common strut. The first connecting strut column includes a plurality of individual first connecting struts that couple the first and second expansion columns. Each first connecting strut has a stair-step geometric configuration. The stair-step geometric configuration includes first and second intermediate sections, a proximal segment coupled to the first expansion column, and a distal segment is a direct extension of an expansion strut of the second expansion column.

The first expansion column and the second expansion column include expansion struts forming a plurality of expansion strut pair loops which couple adjacent expansion struts. Two adjacent expansion strut pairs share a common expansion strut.

### BRIEF DESCRIPTION OF DRAWINGS

Figure 1 shows a side elevation view of an embodiment of a stent, such as a tubular stent.
Figure 2 shows an isometric view of an embodiment of a stent, such as a tubular stent.
Figure 3 shows a cut-open view of an embodiment of a stent. Various expansion columns and connecting strut columns are shown.
Figure 4 shows another cut-open view of an embodiment of a stent.
   Various expansion columns and connecting strut columns are shown.
Figures 5A and 5B show views of expansion struts.
Figures 6A and 6B show more views of expansion struts.
Figure 7A and 7B shows views of connecting struts.
Figure 8 shows a view of conjoining of connecting struts and expansion struts.
Figure 9 shows another view of conjoined connecting struts and expansion struts.
Figure 10 shows another view of conjoined connecting struts and expansion struts.

### DETAILED DESCRIPTION OF DRAWINGS

Some embodiments of stents can be in a state, such as a non-expanded state, a crimped state, and a non-crimped state.

The stents include one or more of a first expansion column, a second expansion column, a first connecting strut column, and can include a third expansion column, and a second connecting strut column.

The first expansion column, the second expansion column, and the third expansion column include individual expansion struts forming a plurality of expansion strut pairs. Two adjacent expansion strut pairs share a common expansion strut.

The first connecting strut column includes a plurality of individual first connecting struts. First connecting struts conjoin the first and second expansion columns. Each first connecting strut has a stair-step geometric configuration. The stair-step configuration of a connecting strut has first and second intermediate sections, a proximal segment coupled to the first expansion column, and a distal segment conjoined directly to an expansion strut of the second expansion column.

The proximal segment of a first connecting strut is coupled to an associated expansion strut in the first expansion column.

The distal segment of a first connecting strut is directly extended from an associated expansion strut in the second expansion column.

At least one of a proximal end and a distal end of a first connecting strut can be a direct extension of an expansion strut pair of the first and second expansion columns.

At least one of a proximal end and a distal end of a first connecting strut in the first expansion column can be coupled to an ipsilateral side of an expansion strut pair of the first and second expansion columns, and at a verticalslant angle to a side of an expansion strut pair of one of the first and second expansion columns.

A longitudinal axis of the distal segment that forms the extension of the associated expansion strut in the second column and a longitudinal axis of the associated expansion strut in the second expansion column are within 20 degrees of each other. The proximal segment of each first connecting strut in the first connecting strut column can be ipsilaterally coupled to an expansion strut pair of the first expansion column and its corresponding distal segment can be ipsilaterally extended from an expansion strut pair of the second expansion column.

A proximal end and a distal end of a first connecting strut in the first connecting strut column can be conjoined on an ipsilateral side of expansion strut pairs of the first and second expansion columns.

Each first connecting strut can have a proximal end that is conjoined to the first expansion column in a first direction, and a distal end that is conjoined to the second expansion column in a second direction that is different from the first direction.

Each first connecting strut has three points of pivot. Each point of pivot can have at least one radius of curvature.

At least a portion of the second intermediate section of a first connecting strut in the first connecting strut column can be positioned in close proximity to a proximal end of an expansion strut pair in the second expansion column. At least a portion of the first intermediate section of a first connecting strut in the first connecting strut column can be in close proximity to a distal end of an expansion strut pair in the first expansion column. Close proximity can be in the range of 0.0254 to 1.27 mm (0.001 to 0.050 of an inch).

Each first connecting strut can have a longitudinal axis that is nonparallel to a longitudinal axis of the stent. The longitudinal axis of each first connecting strut can extend in a first direction that is positioned diagonally relative to the longitudinal axis of the stent. Each first connecting strut can have the same longitudinal axis. All of the first connecting struts in a first connecting strut column can have parallel longitudinal axes. At least a portion of the first connecting struts has asymmetrical geometric configurations.

One expansion strut of an expansion strut pair of the first expansion column can have a stair-step segment at a proximal end. The other expansion strut of the expansion strut pair can have a stair-step segment at a distal end. One expansion strut of an expansion strut pair of the second expansion column can have a stair-step segment at a distal end. The other expansion strut of the expansion strut pair has a stair-step segment at a proximal end.

The first and second intermediate sections of each first connecting strut can be coupled with at least a first radius of curvature. Distal ends of expansion strut pairs of the first expansion column that are coupled to proximal ends of expansion strut pairs of the second expansion column can be vertically offset.

The stent embodiments include a plurality of expansion columns conjoined by a plurality of connecting strut columns.

A plurality of cells is defined by the first expansion column, the second expansion column and the first connecting strut column. The stent cells can have asymmetrical geometries and a quasi-hexagonal geometry in a nominally expanded state.

The second connecting strut column can include a plurality of individual second connecting struts that couple the second and third expansion columns. Each second connecting strut has a stair-step geometric configuration. The geometric configuration has first and second intermediate sections, a proximal segment coupled to the first expansion column, and a distal segment conjoined directly to an expansion strut of the second expansion column.

Each second connecting strut can have a longitudinal axis that is nonparallel to a longitudinal axis of the stent. The longitudinal axis of each second connecting strut can extend in a second direction that is positioned diagonally relative to the longitudinal axis of the stent. Each second connecting strut can have the same longitudinal axis. All of the second connecting struts can have parallel longitudinal axes. Each first connecting strut of the first connecting strut column can have a longitudinal axis that extends in a first direction that is opposite to the second direction of the longitudinal axis of the second connecting struts.

Expansion strut pair loops of the first and second expansion columns or second and third expansion columns can be aligned in a peak-to-valley geometry, a valley-to-peak geometry, or a peak-to-peak geometry.

In some embodiments an expansion column includes six cycles of zigzag form made of twelve expansion strut pairs. Each expansion strut pair includes two expansion struts conjoined by a looped joining section at either a proximal or a distal end. This form of pairing of two expansion struts conjoined by a looped joining section alternates between proximal to distal and distal to proximal, continuing twelve times seamlessly around the circumference of an expansion columns in a cylindrically shaped stent. In some embodiments there can be various expansion struts of different types making up twelve blind-loop expansion strut pairs in an expansion column of a cylindrical stent. In order to have twelve blind-loop expansion strut pairs in an expansion column, there also are twelve looped joining sections, half located in proximal ends and half located in distal ends, in an alternating sequence.

There are various forms of expansion column in a stent, some as depicted in Figure-3. Some embodiments include expansion strut columns with pairs of different expansion struts. Some possible expansion struts include a straight expansion strut shape, a straight part with a short step-down segment at a proximal end and a straight part with short step-up segment at a distal end, a straight part with a short step-up segment at a proximal end and a straight part with short step-down segment at a distal end. Other combinations include a longer straight part with a short step-down segment at a proximal end, a longer straight part with a short step-down segment at a distal end, a longer straight part with a short step-up segment at a proximal end, and a longer straight part with a short step-up segment at a distal end. At each step-up or step-down segment, an expansion strut can have a short-sloped transitional section between the long and short parts. Various combinations are within the scope of the invention as defined in the claims. On both proximal and distal ends of a stent embodiment in Figure-3, a terminating side of an end expansion column can have smooth and evenly rounded loops.

A step-up or step-down segment can be short in length near a proximal or a distal end and can have a short sloped transitional section. A sloped transitional section can provide flexibility, crimping space, and smooth surface modulation effects to the stent performance. One end of a connecting strut can directly conjoin with the long part of an expansion strut at a sloped transitional section of an expansion strut. Another end of a connecting strut can be conjoined to the side of a short step-up or step-down section. One end of a connecting strut can be a direct extension of an expansion strut on the ipsilateral side. The other end can be laterally conjoined on the ipsilateral side of a step short segment of an expansion strut. A connecting strut is an integral part of the stent structure, rather than a separate structure added, welded or attached. Terminology such as expansion strut or connecting strut conveniently describes the structural anatomy and function of various stent portions.

A connecting strut column, including each connecting strut, has stairstep configurations, for example as shown in Figures-4,-7A & -7B,-8,-9 and 10. Due to the stair-step configuration, a longitudinal axis of a connecting strut has a diagonal direction to the vertical or horizontal plane of the stent. A connecting strut of the stent can have, in some embodiments, two horizontal sections, two slanted vertical sections, and three pivot points. The horizontal end of a connecting strut can extend from sloped transitional sections of an expansion strut pair loop. The slanted vertical end can conjoin to an ipsilateral side of an opposed expansion strut pair loop at the step down or step-up segment. A stair-step connecting strut can link an expansion strut pair loop of one expansion column to an expansion strut pair loop of an adjacent expansion column in a vertically split-level linking pathway. Each end of a connecting strut can conjoin on the ipsilateral sides of opposing expansion strut pair loops. A connecting strut that conjoins on ipsilateral sides of expansion strut pair loops, and a split-level linking pathway with multiple pivot points provides stent flexibility,conformability and excellent crimping characteristics to a stent. When each end of a connecting strut is conjoined to a looped pair of expansion struts, the ratio of expansion strut to connecting strut number can be two to one.

When the expansion columns and connecting strut columns are conjoined, the stent can have a continuous, unbroken cylindrical form without breaks or de-linking around the circumference and along the length of a stent. The unbroken link between the expansion and connecting struts can form regular and even asymmetrical cells. The cell size can be maximized or minimized, by programming the dimensions of expansion struts and connecting struts of the stent, as dictated by the clinical or application requirements.

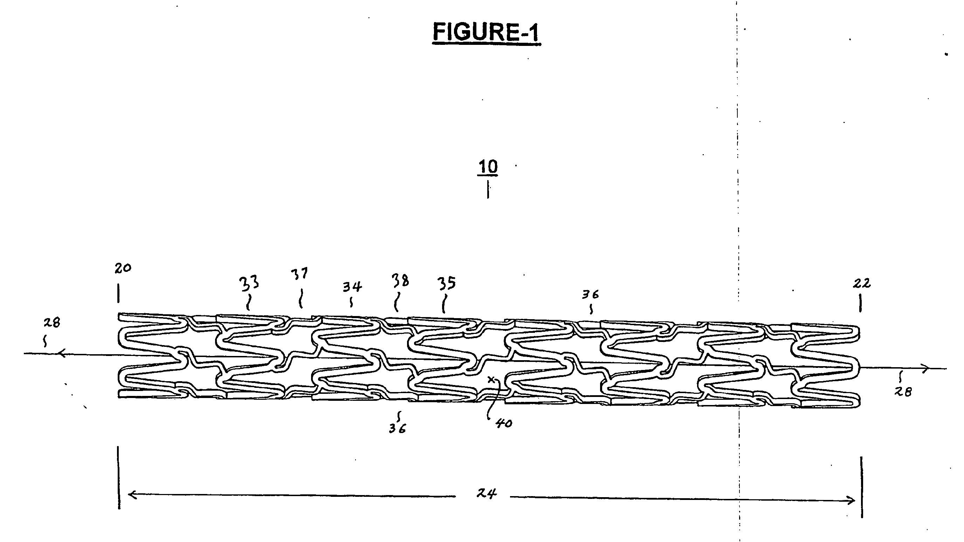

Figure 1 shows one embodiment of a stent 10 in side elevation view. The stent 10 has a proximal end 20 and a distal end 22. The stent 10 can have a tubular or cylindrical structure. The stent 10 can have a longitudinal length 24 and a longitudinal axis 28. Defined by linked connecting and expansion struts are open empty spaces, or cells, for example a cell 40. At both the proximal end 20 and distal end 22 of the stent 10 the terminal ends of expansion strut pairs are evenly rounded for smooth and uniform crimping when the stent 10 is mounted on a delivery balloon.

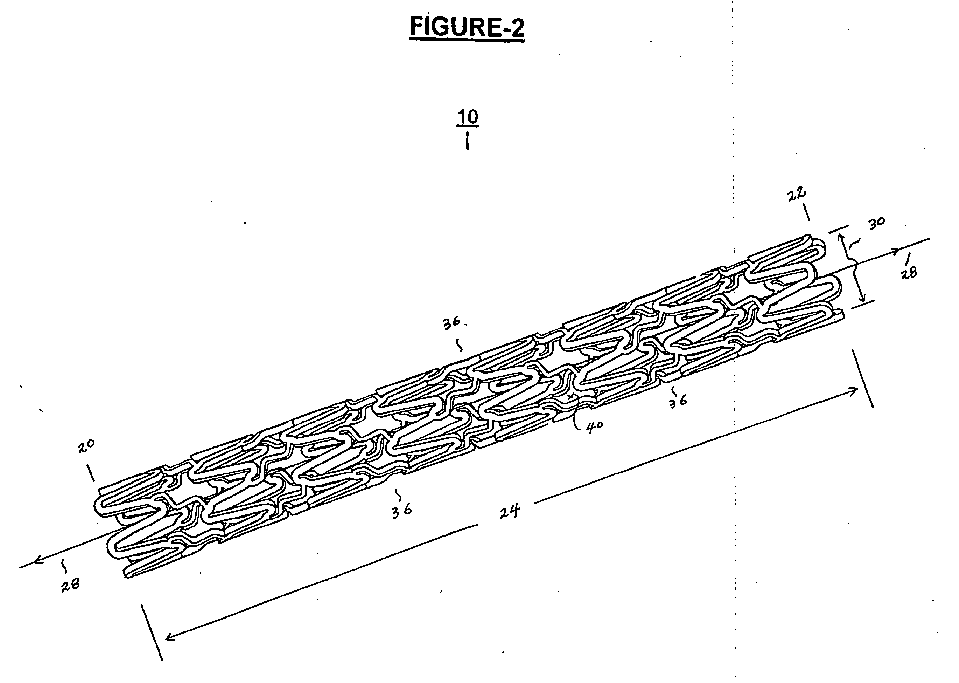

Figure 2 shows an isometric view of an embodiment of a stent, such as a tubular stent. The proximal end 20, distal end 22, the longitudinal axis 28, an internal diameter 30, and a longitudinal length dimension 24 of the cylindrical shape of the stent 10 are shown. The back half of the stent 10 can be seen through cells, such as cell 40.

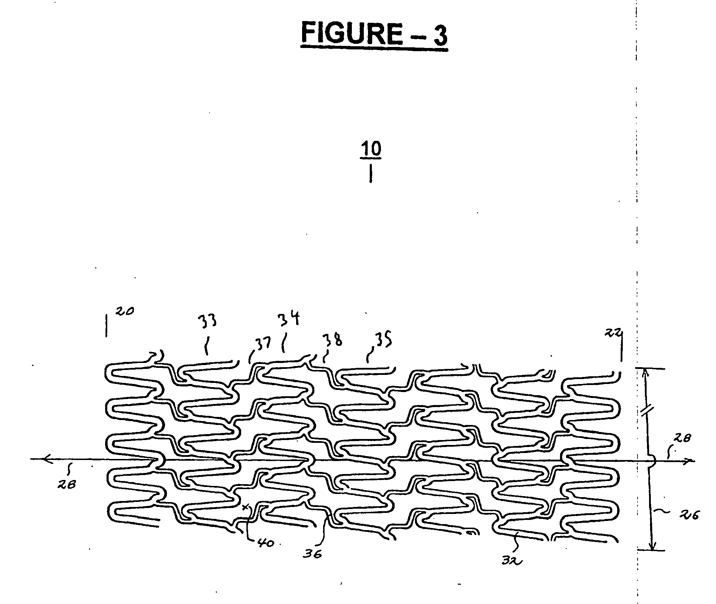

Figure 3 shows a cut-open 2-dimensional view of the cylindrical stent 10. The stent 10 has a longitudinal axis 28, and a circumferential dimension 26. The stent 10 has expansion columns, such as expansion columns 33, 34, and 35 ; and connecting strut columns, such as connecting strut columns 37 and 38. Expansion columns and connecting strut columns alternate in sequence along the longitudinal axis 28 of the stent.

In some embodiment, the expansion columns include a same number of zigzag cycles. Expansion columns shown in Figure-3 have five zigzag cycles. Other embodiments can have more than five cycles, or less than five cycles. In an embodiment with five cycles in expansion columns, there are ten expansion struts. Each cycle includes a pair of expansion struts.

Connecting strut columns have stair-step shaped connecting struts, for example connecting strut 36. For every one pair of expansion struts, there is a connecting strut, making for a ratio of expansion struts to connecting struts of two to one.

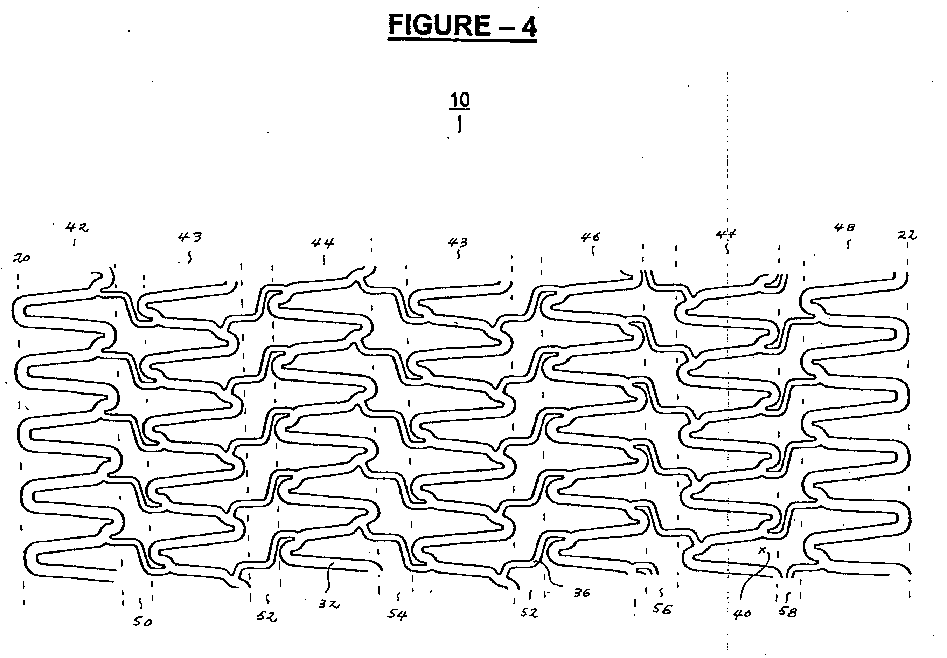

Figure 4 shows expansion columns and connecting strut columns. The embodiment of figure 4 includes a proximal end expansion column 42, a distal end expansion column 48, and expansion columns 43, 44, and 46. In a proximal end expansion column 42, the terminating end loops are rounded in shape making for a smooth surface at the proximal end of the stent 10, suitable for crimping on a delivery balloon. Proximal end expansion column 42 includes an expansion strut type having a straight shape and another expansion strut type having a step-up stair step segment with a sloped-transitional section. These two expansion strut types can make up an expansion strut pair. The distal end expansion strut column 48 can substantially be a mirror image of the proximal end expansion strut column 42. The distal end expansion strut column 48 positions evenly spaced and rounded loops at the terminating end of the stent 10. Evenly spaced and rounded ends can give a smooth surface alignment when the stent 10 is crimped on a delivery balloon.

Expansion column 43 includes different types of expansion strut. One type has a straight strut and the other has a step-up segment at the proximal end and a step-down segment at a distal end. An expansion strut of each type can form an expansion strut pair loop conjoined by a joining strut section, either in the proximal end or the distal end. This pairing can continue around the circumference of the stent 10 in an uninterrupted fashion.

Expansion column 44 includes an expansion strut having a straight shape and another expansion strut with a step-down segment at the proximal end and step-up segment at the distal end. These two types of expansion strut alternate, forming expansion strut pair loops in a continuous manner around the circumference of the cylindrical structure of the stent 10.

Expansion column 46 includes an expansion strut having a step-down segment in the proximal end and an expansion strut having a step-down segment in the distal end. These expansion strut types alternate, forming expansion strut pair loops, in a continuous manner around the circumference.

Figure-4 also shows multiple connecting strut column arrangements. The scope of the invention includes permutations of the expansion column and connecting strut column configurations.

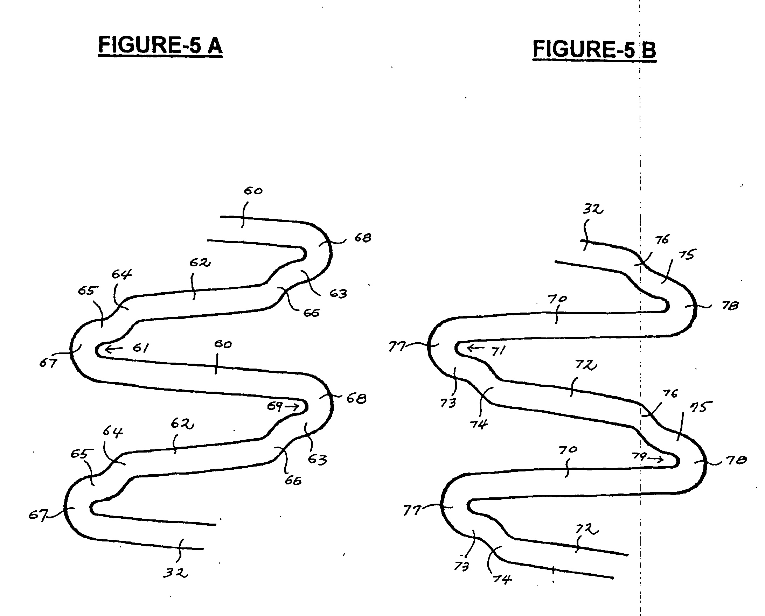

Figure 5A shows expansion struts from expansion column 44. Expansion struts 60 alternate with expansion struts 62, joined either by proximal joining section 67 or distal joining section 68. Proximal joining section 67 defines a proximal cul-de-sac 61. Distal joining section 68 defines a distal cul-de-sac 69. A proximal end of expansion strut 62 includes a step down segment 65 joined by sloped transitional segment 64 to the center of expansion strut 62. A distal end of expansion strut 62 includes a step up segment 63 joined by sloped transitional segment 66 to the center of expansion strut 62.

Figure 5B shows expansion struts from expansion column 43. Expansion struts 70 alternate with expansion struts 72, joined either by proximal joining section 77 or distal joining section 78. Proximal joining section 77 defines a proximal cul-de-sac 71. Distal joining section 78 defines a distal culde-sac 79. A proximal end of expansion strut 72 includes a step up segment 73 joined by sloped transitional segment 74 to the center of expansion strut 72. A distal end of expansion strut 72 includes a step up segment 75 joined by sloped transitional segment 76 to the center of expansion strut 72.

Together, Figures 5A and 5B show the valleys of expansion column 44 aligned with the peaks of expansion column 43.

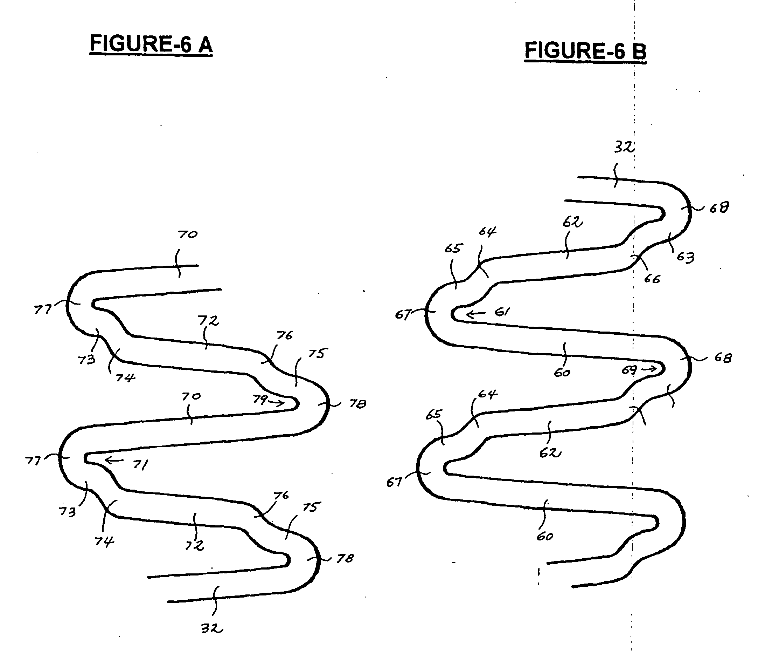

Figure 6A also shows expansion struts of an expansion column 43. Figure 6B also shows expansion struts of an expansion column 44. Together,

Figures 6A and 6B shows the valleys of expansion column 43 aligned with the peaks of expansion column 44.

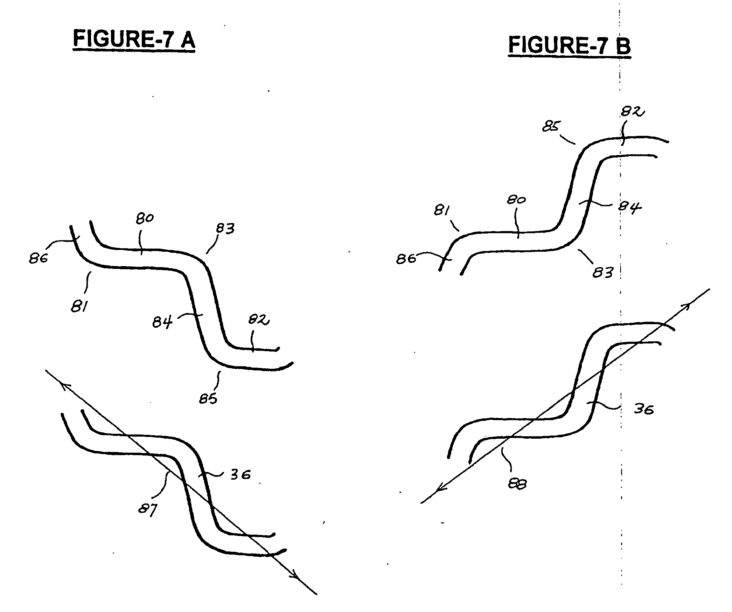

Figure 7A shows connecting struts from connecting strut column 54. A proximal end includes short vertical sloped section 86. Pivot point 81 joins short vertical sloped section 86 with long horizontal section 80. Pivot point 83 joins long horizontal section 80 with long vertical sloped section 84. Pivot point 85 joins long vertical sloped section 84 with short horizontal section 82. Connecting struts in connecting strut column 54 share a longitudinal axis 87.

Figure 7B shows connecting struts from connecting strut column 52, including parts similar to connecting struts from connecting strut column 54. Connecting struts in connecting strut column 52 share a same longitudinal axis 88. The longitudinal axes 87 and 88 are not parallel to each other. Their axes are directed in opposite directions. Figure 7B is a mirror image of Figure 7A.

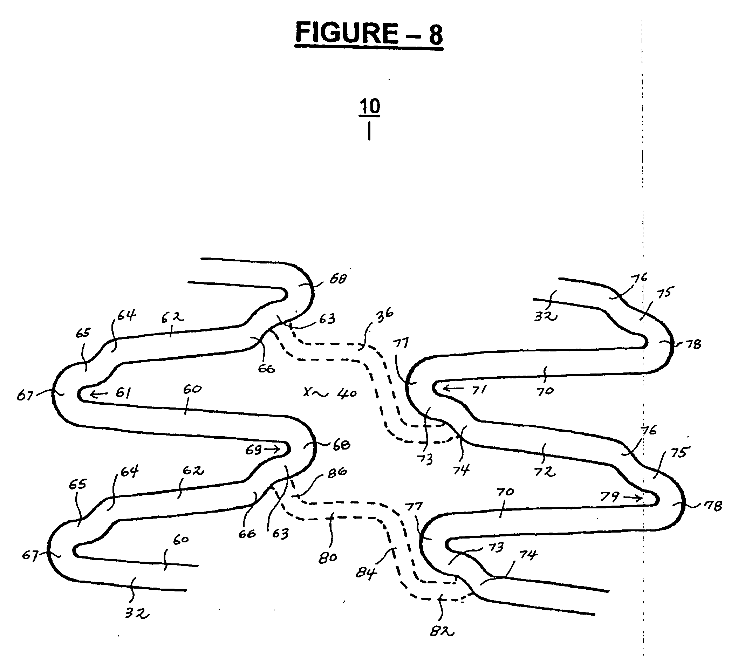

Figure 8 shows the potential conjoining of connecting strut column 54 with expansion strut column 44 on the proximal side of connecting strut column 54 and expansion strut column 43 on the distal side of connecting strut column 54. The step up segment 63 of expansion strut 62 is conjoined to the proximal side of connecting strut column 54. The step up segment 73 of expansion strut 72 is ipsilaterally conjoined to the distal side of connecting strut column 54. The structure of connecting strut column 54 is outlined in dotted lines.

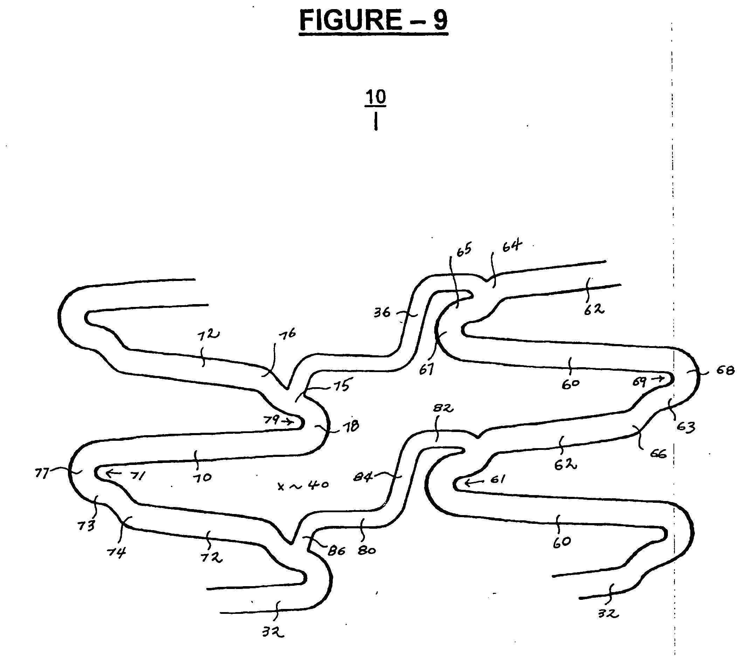

Figure 9 shows the conjoining of connecting strut column 52 with expansion strut column 43 on the proximal side of connecting strut column 52 and expansion strut column 44 on the distal side of connecting strut column 52. The step down segment 75 of expansion strut 72 is conjoined to the proximal side of connecting strut column 52. The step down segment 65 of expansion strut 62 is conjoined to the distal side of connecting strut column 52.

The proximal end 86 of the connecting strut column 52 conjoins on the ipsilateral side to the stepped-down segment 75 of an expansion strut 72. The distal end 82 of a connecting strut column 52 is a direct extension of an expansion strut 62 on the ipsilateral side.

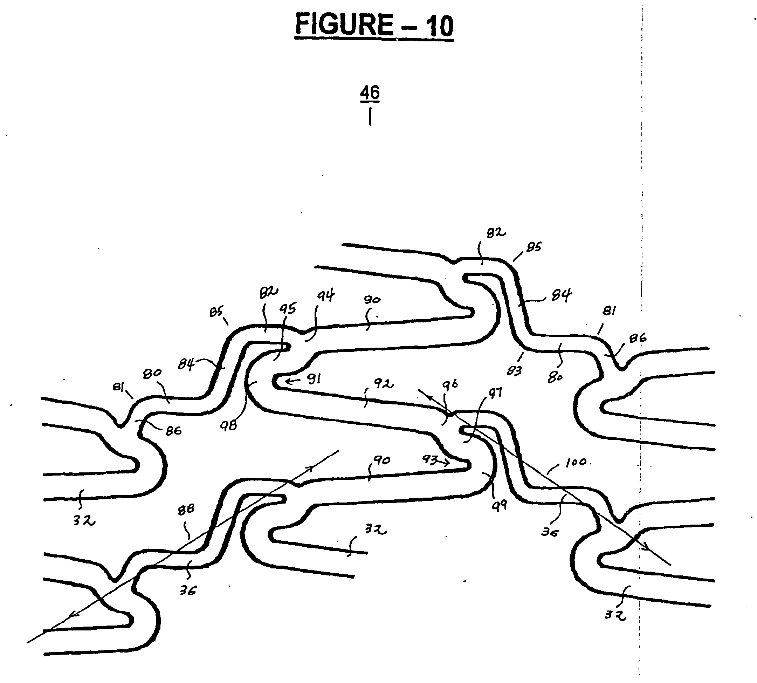

Figure 10 shows the conjoining of expansion column 46 with connecting strut columns 52 and 54. Connecting strut column 52 is conjoined to the proximal side of expansion column 46, and connecting strut column 54 is conjoined to the distal side of expansion strut column 46.

The expansion column 46 shows an important variation of how the connecting struts are conjoined on both proximal and distal ends of the expansion strut pair loops in the expansion strut column 46. Both the proximal expansion strut loops and distal expansion strut loops have direct extensions to the horizontal segment 82 in opposing directions.

## Claims

1. A stent in a non-expanded state, comprising:
a first expansion column (43) including individual expansion struts (70, 72) forming a plurality of expansion strut pair loops that couple adjacent individual expansion struts, wherein two adjacent expansion strut pairs share a common expansion strut;
a second expansion column (44) including individual expansion struts (60, 62) forming a plurality of expansion strut pair loops that couple adjacent individual expansion struts, wherein two adjacent expansion strut pairs share a common expansion strut and one expansion strut (62) of an expansion strut pair has a stair-step segment (65) at a proximal end, the expansion strut (62) with the stair-step segment (65) having a straight part with a short step-up section or a short step-down section (65) at a proximal end and a short sloped transitional section (64); and
a first connecting strut column (52) including a plurality of individual connecting struts (36), wherein each of an individual first connecting strut (36) has a stair-step geometric configuration with first and second intermediate sections (80, 84), a proximal segment (86) coupled to the first expansion column (43) and a distal segment (82) conjoined directly to an expansion strut (62) of the second expansion column, **characterized in that** at least some of the connecting struts (36) have a distal end (82) conjoined directly with the straight part of an expansion strut (62) at a short sloped transitional section (64).

2. The stent of claim 1, wherein at least one of a proximal and distal end of a first connecting strut (36) is a direct extension of one of an expansion strut pair loop of the first and second expansion columns (43, 44).

3. The stent of claim 1, wherein at least one of a proximal and distal end of a first connecting strut (36) is coupled to a side of an expansion strut pair loop of one of the first and second expansion columns (43, 44).

4. The stent of claim 1, wherein at least one of a proximal and distal end of a first connecting strut (36) is coupled at a vertical-slant angle to a side of an expansion strut pair loop of one of the first and second expansion columns (43, 44).

5. The stent of claim 1, wherein a longitudinal axis of the distal segment (82) that forms the extension of the associated expansion strut (62) in the second column (44) and a longitudinal axis of the associated expansion strut (62) in the second expansion column (44) are within 20 degrees of each other.

6. The stent of claim 1, wherein the proximal segment (86) of each first connecting strut (36) in the first connecting strut column (52) is coupled to an expansion strut pair of the first expansion column (43) and its corresponding distal segment (82) is ipsilaterally extended from an expansion strut pair loop of the second expansion column (44).

7. The stent of claim 1, wherein each of a first connecting strut (36) in the first connecting strut column (52) has a proximal end that is conjoined to the first expansion column (43) in a first direction, and a distal end that is conjoined to the second expansion column (44) in a second direction that is opposite to the first direction.

8. The stent of claim 7, wherein a proximal end and a distal end of a first connecting strut (36) in the first connecting strut column (52) are conjoined on an ipsilateral side of expansion strut pairs of the first and second expansion columns (43, 44).

9. The stent of claim 1, wherein each first connecting strut (36) of the first connecting strut column (52) has three points of pivot (81, 83, 85).

10. The stent of claim 10, wherein each point of pivot (81, 83, 85) has at least one radius of curvature.

11. The stent of claim 1, further comprising:
a third expansion column (46) including individual expansion struts forming a plurality of expansion strut pairs, wherein two adjacent expansion strut pairs share a common strut;
a second connecting strut column (56) including a plurality of individual second connecting struts that couple the second and third expansion columns, wherein each of an individual second connecting strut has a stair-step geometric configuration with first and second intermediate sections (80, 84), a proximal segment coupled to the second expansion column (44) and a distal segment conjoined directly to an expansion strut of the third expansion column (46).

12. The stent of claim 1, wherein expansion strut pair loops of the first and second expansion columns (43, 44) are aligned in a peak-to-valley geometry.

13. The stent of claim 1, wherein expansion strut pair loops of the first and second expansion columns (43, 44) are aligned in a valley-to-peak geometry.

14. The stent of claim 1, wherein expansion strut pair loops of the first and second expansion columns (43, 44) are aligned in a peak-to-peak geometry.

## Patentansprüche

1. Stent in einem nicht expandierten Zustand, umfassend:
eine erste Expansionssäule (43), umfassend einzelne Expansionsstreben (70, 72), die eine Mehrzahl Expansionsstrebenpaarschleifen bilden, die benachbarte einzelne Expansionsstreben koppeln, wobei zwei benachbarte Expansionsstrebenpaare eine gemeinsame Expansionsstrebe teilen;
eine zweite Expansionssäule (44), umfassend einzelne Expansionsstreben (60, 62), die eine Mehrzahl Expansionsstrebenpaarschleifen bilden, die benachbarte einzelne Expansionsstreben koppeln, wobei zwei benachbarte Expansionsstrebenpaare eine gemeinsame Expansionsstrebe teilen und eine Expansionsstrebe (62) eines Expansionsstrebenpaars ein Stufensegment (65) an einem proximalen Ende aufweist, wobei die Expansionsstrebe (62) mit dem Stufensegment (65) einen geraden Teil mit einem kurzen Aufwärtsstufenabschnitt oder einem kurzen Abwärtsstufenabschnitt (65) an einem proximalen Ende und einem kurzen geneigten Übergangsabschnitt (64) aufweist; und
eine erste Verbindungsstrebensäule (52), umfassend eine Mehrzahl einzelner Verbindungsstreben (36), wobei jede der einzelnen ersten Verbindungsstreben (36) eine stufengeometrische Ausgestaltung mit ersten und zweiten Zwischenabschnitten (80, 84), aufweist, wobei ein proximales Segment (86) an die erste Expansionssäule (43) gekoppelt ist und ein distales Segment (82) direkt mit einer Expansionsstrebe (62) der zweiten Expansionssäule verbunden ist, **dadurch gekennzeichnet, dass** mindestens einige der Verbindungsstreben (36) ein digitales Ende (82) aufweisen, das direkt mit dem geraden Teil einer Expansionsstrebe (62) an einem kurzen geneigten Übergangsabschnitt (64) verbunden sind.

2. Stent nach Anspruch 1, wobei mindestens eines eines proximalen und distalen Endes einer ersten Verbindungsstrebe (36) eine direkte Verlängerung einer einer Expansionsstrebenpaarschleife der ersten und zweiten Expansionssäulen (43, 44) ist.

3. Stent nach Anspruch 1, wobei mindestens eines eines proximalen und distalen Endes einer Verbindungsstrebe (36) an eine Seite einer Expansionsstrebenpaarschleife einer der ersten und zweiten Expansionssäule (43, 44) gekoppelt ist.

4. Stent nach Anspruch 1, wobei mindestens eines eines proximalen und distalen Endes einer ersten Verbindungsstrebe (36) in einem vertikal schrägen Winkel zu einer Seite einer Expansionsstrebenpaarschleife einer der ersten und zweiten Expansionssäule (43, 44) gekoppelt ist.

5. Stent nach Anspruch 1, wobei eine Längsachse des distalen Segments (82), das die Verlängerung der zugehörigen Expansionsstrebe (62) in der zweiten Säule (44) bildet, und eine Längsachse der zugehörigen Expansionsstrebe (62) in der zweiten Expansionssäule (44) innerhalb von 20° zueinander liegen.

6. Stent nach Anspruch 1, wobei das proximale Segment (86) jeder ersten Verbindungsstrebe (36) in der ersten Verbindungsstrebensäule (52) an ein Expansionsstrebenpaar der ersten Expansionssäule (43) gekoppelt ist, und sich sein entsprechendes distales Segment (82) gleichseitig von einer Expansionsstrebenpaarschleife der zweiten Expansionssäule (44) erstreckt.

7. Stent nach Anspruch 1, wobei jede einer ersten Verbindungsstrebe (36) in der ersten Verbindungsstrebensäule (52) ein proximales Ende aufweist, das mit der ersten Expansionssäule (43) in einer ersten Richtung verbunden ist, und ein distales Ende, das mit der zweiten Expansionssäule (44) in einer zweiten Richtung verbunden ist, die entgegengesetzt zur ersten Richtung ist.

8. Stent nach Anspruch 7, wobei ein proximales Ende und ein distales Ende einer ersten Verbindungsstrebe (36) in der ersten Verbindungsstrebensäule (52) an einer gleichliegenden Seite von Expansionsstrebenpaaren der ersten und zweiten Expansionssäulen (43, 44) verbunden sind.

9. Stent nach Anspruch 1, wobei jede erste Verbindungsstrebe (36) der ersten Verbindungsstrebensäule (52) drei Schwenkpunkte (81, 83, 85) aufweist.

10. Stent nach Anspruch 10, wobei jeder Schwenkpunkt (81, 83, 85) mindestens einen Krümmungsradius aufweist.

11. Stent nach Anspruch 1, überdies umfassend:
eine dritte Expansionssäule (46), umfassend einzelne Expansionsstreben, die eine Mehrzahl Expansionsstrebenpaare bilden, wobei zwei benachbarte Expansionsstrebenpaare eine gemeinsame Strebe teilen;
eine zweite Verbindungsstrebensäule (56), umfassend eine Mehrzahl einzelner zweiter Verbindungsstreben, die die zweiten und dritten Expansionssäulen koppeln, wobei jede der einzelnen zweiten Verbindungsstreben eine stufengeometrische Ausgestaltung mit ersten und zweiten Zwischenabschnitten (80, 84) aufweist, ein proximales Segment, das an die zweite Expansionssäule (44) gekoppelt ist, und ein distales Segment, das direkt mit einer Expansionsstrebe der dritten Expansionssäule (46) verbunden ist.

12. Stent nach Anspruch 1, wobei Expansionsstrebenpaarschleifen der ersten und zweiten Expansionssäulen (43, 44) in einer Spitze-Tal-Geometrie ausgerichtet sind.

13. Stent nach Anspruch 1, wobei Expansionsstrebenpaarschleifen der ersten und zweiten Expansionssäulen (43, 44) in einer Tal-Spitze-Geometrie ausgerichtet sind.

14. Stent nach Anspruch 1, wobei Expansionsstrebenpaarschleifen der ersten und zweiten Expansionssäulen (43, 44) in einer Spitze-Spitze-Geometrie ausgerichtet sind.

## Revendications

1. Stent dans un état non dilaté, comprenant :
une première colonne de dilatation (43) comprenant des supports de dilatation (70, 72) individuels formant une pluralité de boucles de paires de supports de dilatation qui accouplent des supports de dilatation individuels adjacents, où deux paires de supports de dilatation adjacentes partagent un support de dilatation commun ;
une deuxième colonne de dilatation (44) comprenant des supports de dilatation (60, 62) individuels formant une pluralité de boucles de paires de supports de dilatation qui accouplent des supports de dilatation individuels adjacents, où deux paires de supports de dilatation adjacentes partagent un support de dilatation commun, et où un support de dilatation (62) d'une paire de supports de dilatation a un segment en marches d'escalier (65) à une extrémité proximale, le support de dilatation (62) avec le segment en marches d'escalier (65) ayant une partie rectiligne avec une courte section de montée ou une courte section de descente (65) à une extrémité proximale, et une courte section de transition inclinée (64) ; et
une première colonne de support de raccordement (52) comprenant une pluralité de supports de raccordement (36) individuels, où chaque premier support de raccordement (36) individuel a une configuration géométrique en marches d'escalier avec des première et deuxième sections intermédiaires (80, 84), un segment proximal (86) couplé à la première colonne de dilatation (43), et un segment distal (82) réuni directement à un support de dilatation (62) de la deuxième colonne de dilatation, **caractérisé en ce que** au moins certains des supports de raccordement (36) ont une extrémité distale (82) réunie directement à la partie rectiligne d'un support de dilatation (62) au niveau d'une courte section de transition (64) en pente.

2. Stent selon la revendication 1, où au moins une extrémité parmi une extrémité proximale et une extrémité distale d'un premier support de raccordement (36) est un prolongement direct d'une boucle de paires de supports de dilatation des première et deuxième colonnes de dilatation (43, 44).

3. Stent selon la revendication 1, où au moins une extrémité parmi une extrémité proximale et une extrémité distale d'un premier support de raccordement (36) est raccordée à un côté d'une boucle de paires de supports de dilatation d'une des première et deuxième colonnes de dilatation (43, 44).

4. Stent selon la revendication 1, où au moins une extrémité parmi une extrémité proximale et une extrémité distale d'un premier support de raccordement (36) est couplée, sous un angle d'inclinaison vertical, à un côté d'une boucle de paires de supports de dilatation d'une colonne parmi les première et deuxième colonnes de dilatation (43, 44).

5. Stent selon la revendication 1, où un axe longitudinal du segment distal (82) qui forme le prolongement du support de dilatation (62) associé dans la deuxième colonne (44), et un axe longitudinal du support de dilatation (62) associé dans la deuxième colonne de dilatation (44) forment l'un par rapport à l'autre un angle ne dépassant pas 20 degrés.

6. Stent selon la revendication 1, où le segment proximal (86) de chaque premier support de raccordement (36) dans la première colonne de support de raccordement (52) est couplé à une paire de supports de dilatation de la première colonne de dilatation (43), et où son segment distal (82) correspondant est prolongé de façon ipsilatérale à partir d'une boucle de paires de supports de dilatation de la deuxième colonne de dilatation (44).

7. Stent selon la revendication 1, où chacun d'un premier support de raccordement (36) dans la première colonne de support de raccordement (52) a une extrémité proximale qui est réunie à la première colonne de dilatation (43) dans une première direction, et une extrémité distale qui est réunie à la deuxième colonne de dilatation (44) dans une deuxième direction qui est opposée à la première direction.

8. Stent selon la revendication 7, où une extrémité proximale et une extrémité distale d'un premier support de raccordement (36) dans la première colonne de support de raccordement (52) sont réunies sur un côté ipsilatéral de paires de supports de dilatation des première et deuxième colonnes de dilatation (43, 44).

9. Stent selon la revendication 1, où chaque premier support de raccordement (36) de la première colonne de support de raccordement (52) a trois points de pivot (81, 83, 85).

10. Stent selon la revendication 10, où chaque point de pivot (81, 83, 85) a au moins un rayon de courbure.

11. Stent selon la revendication 1, comprenant également :
une troisième colonne de dilatation (46) comprenant des supports de dilatation individuels formant une pluralité de paires de supports de dilatation, où deux paires de supports de dilatation adjacentes partagent un support commun ;
une deuxième colonne de support de raccordement (56) comprenant une pluralité de deuxièmes supports de raccordement individuels qui accouplent les deuxième et troisième colonnes de dilatation, où chacun d'un deuxième support de raccordement individuel a une configuration géométrique en marches d'escalier avec des première et deuxième sections intermédiaires (80, 84), un segment proximal couplé à la deuxième colonne de dilatation (44), et un segment distal réuni directement à un support de dilatation de la troisième colonne de dilatation (46).

12. Stent selon la revendication 1, où les boucles de de paires de supports de dilatation des première et deuxième colonnes de dilatation (43, 44) sont alignées dans une géométrie de crête à creux.

13. Stent selon la revendication 1, où les boucles de de paires de supports de dilatation des première et deuxième colonnes de dilatation (43, 44) sont alignées dans une géométrie de creux à crête.

14. Stent selon la revendication 1, où les boucles de de paires de supports de dilatation des première et deuxième colonnes de dilatation (43, 44) sont alignées dans une géométrie de crête à crête.
